# EUROPEAN PATENT APPLICATION

(11) **EP 2 893 887 A1**
(43) Date of publication of application: **15.07.2015**
(21) Application number: 14150806.9
(22) Date of filing: 10.01.2014
(51) Int. Cl.: A61B 17/34

(54) **An indication device for locating a natural cavity in a body**

(71) Applicant: University College Cork, Cork (IE)
(72) Inventor: O'Shea, Conor, County Kerry (IE); Lee, Peter, County Cork (IE); Cantillon-Murphy, Padraig, Cork (IE); Keane, John, County Kerry (IE); Keohane, Kate, County Cork (IE); Delaney, John, Cork (IE); MacSuibhne, Peadar, Co. Chorcai (IE)
(74) Representative: O'Neill, Brian

(57) **Abstract**

The present invention provides a device for indicating the location of a natural cavity in a body, the device comprising, in a preferred embodiment, a piston and cylinder type syringe for the immediate detection of a natural cavity in a multicellular organism by means of utilising the haptic feedback associated with the loss of resistance technique, whilst also providing a visual indication which forms an integrated part of the syringe, to confirm successful needle placement in the desired intracorporeal cavity. Also a method of detecting and locating the distal tip of a probe such as a needle in the natural cavity is described.

## Description

### Field of the invention

The present invention relates to an indication device for, and method of, signalling the correct location or entry of a probe such as an epidural needle or the like into a natural cavity in a body through both visual and tactile feedback, for example when positioning the distal tip of such a needle in the natural cavity of the epidural space.

### Background of the invention

Epidural administration of local anaesthetic and/or opioids is performed for analgesia and anaesthesia in the perioperative period or to provide analgesia before surgical procedures and labour. It is also commonly used as a therapeutic method for pain relief. Epidurals block the nerve impulses from certain spinal segments resulting in decreased sensation in the required part of the body. Although epidural anaesthesia has been part of anaesthetic practice since 1901, localisation of the epidural space remains technically difficult. Epidural analgesia has been described as the gold standard of pain control. The technique requires a clinician to blindly pass a needle through soft tissue and ligament between the caudally protruding spinous processes of the vertebrae. Confirmation of needle tip entry to the epidural space is most commonly achieved by the loss-of-resistance (LOR) technique. The preferred method of carrying out the LOR technique involves inserting a Tuohy needle into the interspinous ligament and then attaching a syringe filled with saline or air. The saline solution or air provides resistance feedback to the operator as the needle moves toward the epidural space. The needle is grasped with the nondominant hand and is advanced toward the epidural space while the dominant hand (thumb) applies either a constant steady pressure or a fluctuating/pulsing pressure to the syringe plunger. There is a gradual increase in resistance until the epidural space is reached, whereby the resistance will drop to zero. The epidural space is located immediately beyond the ligamentum flavum and before the dura. Conventional LOR techniques rely exclusively on the tactile sensation. When the epidural space has been reached, the pressure on the plunger injects saline/air into the space and further needle advancement is discontinued. Care must be taken not to advance the needle any further once the epidural space has been reached. Once the operator detects the LOR and is satisfied that the Tuohy needle is in place, the syringe is removed and a catheter is threaded through the needle into the epidural space. The needle is then carefully removed, leaving the catheter in place to provide medication either through periodic injections or by continuous infusion. The catheter is taped to the patient's back to prevent it from slipping out.

Incorrect epidural placement can lead to a number of undesired consequences. The LOR is subtle and may be difficult to detect especially for trainee anaesthetists.

Complications associated with the LOR technique include pneumocephalus, spinal cord and nerve root compression, subcutaneous emphysema, venous air embolism, neurologic injury and post-dural puncture headache. The latter is caused by inadvertent puncture of the dura following failure to identify the epidural space. The incidence of dural puncture is approximately 1 % of all epidurals and approximately 80-86% of occasions of dural puncture will lead to a post dural puncture headache. The incidence is dependent on clinician experience reflecting the importance of technique and a three-dimensional understanding and tactile appreciation of anatomy. For trainee anaesthetists, the mean (95% confidence intervals) epidural failure rate is one in every five consecutive epidurals for the first 50 epidurals performed. An appreciation of the feel of the loss-of-resistance technique is difficult to teach or demonstrate. As much as 75 attempts at epidural anaesthesia may be required before a 90% success rate is achieved. The technique may also be unreliable in patients with altered vertebral anatomy or calcified spinal ligaments, while epidural anaesthesia failure rates are greater in obese patients. Another more common complication with current epidural administration techniques is that of false positive infusions whereby the user experiences a loss of haptic feedback and assumes the epidural space is reached. This is more common in obese patients as there may be some leakage as the epidural needle passes through layers of fatty tissue giving false LOR feed back.

A second clinical application of the present invention is identifying the peritoneal cavity during laparoscopic surgery. Laparoscopy is a technique that allows surgery to be performed through a number of small incisions and is quickly becoming the gold standard in the treatment of malignancies arising in the abdomen. Laparoscopy requires the identification of the peritoneal cavity to create a pneumoperitoneum through insufflation. The current method requires passing a Veress needle through the abdominal layers until the user deems they have reached the peritoneal cavity. However, this can often take several attempts as it is difficult to discern between the different layers of tissue due to the sharpness of the Veress needle. Similar to locating the epidural space, a pressure differential method may be used to confirm accurate placement of a needle tip in the peritoneal cavity.

A third clinical application of the present invention is in percutaneous endoscopic gastrostomy (PEG) tube placement. PEG is an endoscopic medical procedure in which a tube (PEG tube) is passed into a patient's stomach through the abdominal wall, most commonly to provide a means of feeding when oral intake is not adequate (for example, because of dysphagia or sedation). This provides enteral nutrition (making use of the natural digestion process of the gastrointestinal tract) despite bypassing the mouth; enteral nutrition is generally preferable to parenteral nutrition (which is only used when the gastrointestinal tract must be avoided). The PEG procedure is an alternative to open surgical gastrostomy insertion, and does not require a general anaesthetic; mild sedation is typically used. PEG tubes may also be extended into the small intestine by passing a jejunal extension tube (PEG-J tube) through the PEG tube and into the jejunum via the pylorus. In the case of both PEG and PEG-J tube placement, the procedure begins with the percutaneous insertion of a small needle across the skin and fatty layers into the gastrointestinal tract. Similar to locating the epidural space, a pressure differential method may be used to confirm accurate placement of a needle tip in the stomach or gastrointestinal tract.

A fourth application of the present invention is in placing bladder catheters. Urinary obstruction or failure to void is a common condition, particularly in older men. In some cases where catheterization of the urethra is problematic or impossible, a physician may decide to insert the bladder catheter through the abdominal wall to get to the urinary tract. After the proposed placement position of the catheter is determined, a small needle will be inserted into the skin, and then the catheter is threaded through the opening inside the needle called the lumen. A percutaneous catheter sheath may be used to guide the catheter to the bladder without damaging any of the surrounding tissue. The loose end of the catheter is then taped securely to the skin. In some such cases, percutaneous nephrostomy may be required, when an obstruction exists whereby urine is unable to pass from the kidney to the bladder. In these cases, a small drainage catheter is inserted into the patient's kidney. The tube enters the skin in the side of the lower back to drain urine from the kidney and is initially placed by percutaneous insertion of a small needle under image guidance. Similar to locating the epidural space, a pressure differential method may be used to confirm accurate placement of a needle tip in the kidney or bladder in the scenarios outlined above.

A fifth application of the present invention is in percutaneous abscess drainage. An abscess is an infected fluid collection within the body. In general, people who have an abscess will experience fever, chills and pain in the approximate location of the area that is involved. If a patient has these symptoms, it is not uncommon that they will undergo an imaging test, (usually a CT scan or an ultrasound), to assist in identifying and making the correct diagnosis of an abscess. Once the diagnosis of an abscess has been made, your physician and an interventional radiologist will work together to decide the appropriate therapy. As long as it is deemed safe, percutaneous abscess drainage offers a minimally invasive therapy that can be used to treat the abscess. In percutaneous abscess drainage, an interventional radiologist uses imaging guidance (CT, ultrasound or fluoroscopy) to place a thin needle into the abscess to remove or drain the infected fluid from an area of the body such as the chest, abdomen or pelvis. As in the previous applications, a pressure differential method may be used to confirm accurate placement of a needle tip in an endoluminal abscess.

A number of apparatuses have been developed based on pressure differential to assist in identifying the epidural space. U.S. Patent No. 7,175,608 discloses a device which visually determines when the needle tip enters the epidural space. The device comprises a chamber capable of connection to an epidural needle with a non-return valve on its proximal end and a slip luer lock on its distal end and also comprises an inflatable diaphragm. The chamber is pressurised prior to advancing the needle towards the epidural space thus inflating the diaphragm. As the needle breaches the ligamentum flavum, the pressure difference deflates the diaphragm and gives a visual indication that the epidural space has been reached. However, this apparatus completely removes the haptic feedback associated with the LOR technique and replaces the tactile with a visual confirmation. This presents possible complications if fibrous tissue lodges in the needle as it advances and prevents the diaphragm from deflating. Conversely the chamber may leak air or saline into the fatty tissue as it advances and in doing so slowly deflates the diaphragm providing a false confirmation of location.

U.S. Patent No. 5,902,273 discloses a device that uses a visual pressure indicator, comprising a bellows and spring loaded piston to detect the drop in positive pressure as the needle enters the epidural space. This device also comprises a one-way valve for positive pressure build-up which displaces the piston vertically upwards. As the needle tip enters the epidural space, the positive pressure in the chamber drops and the indicator will return to its original position due to the elastic force of the spring. This device replaces the haptic feedback associated with the LOR technique and relies solely on the effectiveness of the visual indicator to immediately collapse on localisation of the epidural space. This complex assembly cannot be calibrated for varying pressures associated with thin and obese patients and in practice may fail due to mechanical stickiness.

Therefore an object of the invention is to deliver a lightweight, durable and easy to use indication device and method that improves the reliability and effectiveness of locating a natural cavity in the body such as the epidural space for epidural anaesthesia or analgesia.

Another object of this invention is to provide a natural cavity location indication device with an integrated visual pressure change indicator to immediately signify when the space has been reached whilst retaining the capability to utilise the conventional loss of resistance technique.

Another object of this invention, in the case of epidural administration, is to provide a natural cavity location indication device with enough positive pressure to penetrate the ligamentum flavum and deflect the dura tissue upon introduction of the needle tip into the epidural space preventing dural puncture, without injecting excessive amounts of fluid. In addition, the positive pressure prevents tissue from lodging in the needle as it passes through the interspinous ligament.

Another object of this invention is to provide a natural cavity location indication device with an objective and effective change of pressure visual indicator to both the user and the observer for ease of use and training purposes.

Another object of this invention is to provide a natural cavity location indication device which can be pressurised with either a gas such as air or a liquid such as saline and having a pressure applicator in the form of a plunger which may be manually advanced by either a constant pressure or pulsing pressure LOR technique, and may be re-pressurised when pressure is lost, for example in soft loose connective tissue, as it is advanced towards the natural cavity.

Another object of this invention is to provide a low cost natural cavity location indication device which is easily manufactured with plastic or a similar material, replacing the more expensive glass epidural syringes.

A further object of the invention is to provide an indication device capable of identifying a body or organ cavity, such as the spinal cavity, cranial cavity, ventral cavity, gastrointestinal tract, bladder cavity, kidney cavity or abscess which includes similar steps to locating the epidural space, namely pressurising a device and displacing a visual indicator, and advancing said indication device with an attached needle whilst employing the LOR technique until said cavity is located.

### Summary of the invention

These and other objects of the invention as will be apparent herein are accomplished by providing, according to a first aspect of the invention, an indication device which provides both visual and haptic feedback in signalling entry of a probe into a natural cavity, the device comprising;
a pressurisable chamber having at least an outlet from which a working fluid may be dispensed;
a manually actuatable pressure applicator operable to alter the pressure within the chamber; and a visual pressure change indicator in fluid communication with the chamber and comprising at least one pressure expandable diaphragm.

Preferably, the at least one expandable diaphragm is arranged to bulge outwards to signify when the chamber is suitably pressurised.

Preferably, the visual pressure change indicator comprises an enclosure having an entry in fluid communication with the chamber, and an opening over which the at least one expandable diaphragm provides a fluid tight covering.

The perimeter of the diaphragm may be secured between an uppermost part of the enclosure and a cap which is secured to a top of the enclosure and defines the opening. An O-ring may also be positioned between the cap and the enclosure to further secure the diaphragm in place.

Preferably the diaphragm is circular in shape and made of an elastomeric or other suitable material.

Preferably, the visual pressure change indicator is adapted to change colour in response to a predefined pressure change within the chamber.

Preferably, the visual pressure change indicator comprises a first diaphragm having one or more apertures therein; and a second diaphragm underlying the first diaphragm and adapted to protrude through the one or more apertures in said upper diaphragm when the chamber is suitably pressurised.

Preferably, the first and second diaphragms are of contrasting colour.

The second or lower membrane is hidden from view by the first or upper membrane when the device is in an equilibrium state. When the enclosure of the visual indicator is pressurised, the lower membrane, which comprises a different, contrasting colour relative to the upper membrane, bulges upwards through the openings in the upper membrane. Once the endoluminal cavity has been reached, the drop in pressure returns the protruding lower membrane to its secluded position.

In another embodiment, a change in colour of the visual indicator occurs as the diaphragm bulges outwards. In the equilibrium state, the diaphragm comprises a very apparent solid colour. As the diaphragm bulges outwards and the thickness of the diaphragm reduces, the apparent colour is replaced with a transparent finish to the diaphragm. When the diaphragm contracts, said diaphragm returns to its original solid colour.

Preferably, the outlet defines a connection port adapted to receive a hollow probe in fluid communication with the chamber.

The connection port may take the form of a luer slip or luer lock connection or a specific connection required for epidural administration.

Preferably, the chamber is defined by a syringe barrel.

Preferably, the pressure change indicator is formed integrally with the syringe barrel.

Preferably, the indication device comprises a handle on the syringe barrel.

Preferably, the pressure applicator comprises a plunger slidably received within the syringe barrel.

Preferably, the plunger is adapted to operate as a loss of resistance plunger.

The indication device is pressurised with a fluid by means of advancing the plunger relative to the syringe barrel. The term fluid may refer to a gas such as air or a liquid. In the preferred embodiment, the device is adapted for use with either air or a saline solution. The plunger is manually advanced with the user's thumb by either a constant pressure or pulsing pressure technique until the diaphragm bulges outwards signalling that the chamber has been adequately pressurised. The user is equipped with both the haptic feedback from the plunger and the visual feedback from the diaphragm to confirm immediate placement of the probe in the desired natural cavity or space. Due to the incompressible nature of saline, the diaphragm tends to bulge immediately on advancement of the plunger until a certain volume of the saline, for example approximately 0.5 ml, is displaced at which point the desired bulge in the diaphragm is achieved. For air or other suitable gas, in an exemplary embodiment approximately 2.5 ml may be displaced to achieve the same diaphragm displacement. It will of course be understood that these values are subject to change depending on the application.

In an embodiment for epidural administration, the enclosure of the visual indicator may preferably be charged with sufficient positive pressure to penetrate the ligamentum flavum and deflect the dura tissue upon introduction of the needle tip into the epidural space preventing dural puncture, without injecting excessive amounts of fluid. In addition, the positive pressure prevents tissue from lodging in the probe as it passes through the interspinous ligament. The pressure required to displace the diaphragm may range from 25 to 400 mmHg depending on the application.

Preferably, the indication device comprises a stop operable to limit the axial displacement of the plunger within the syringe barrel.

Preferably, the internal diameter of said syringe barrel is no less than 5 mm and no more than 20 mm.

According to a second aspect of the invention there is provided a method of indicating the location of a natural cavity comprising the steps of:
pressurising a chamber having at least an outlet from which a working fluid may be dispensed through a probe secured in fluid communication to the outlet by manually actuating a pressure applicator in operative association with the chamber;
advancing the probe transcutaneously through tissue towards the natural cavity while applying a compressive force to the pressure applicator until a visual change occurs in a visual pressure change indicator in fluid communication with the chamber and a loss in resistance occurs to the application of force to the pressure applicator as the probe enters the cavity.

In an alternative embodiment of the indication device the diaphragm comprises an inflatable balloon which envelopes a distal end of the syringe barrel. A number of inflation holes are cut into the surface of the barrel beneath the balloon arranged in a polar array extending around the circumference of the barrel. When the plunger is advanced and the needle experiences resistance as it passes through tissue, the balloon inflates radially outwards due to the increased pressure. As the needle tip reaches the space, the balloon collapses due to the drop in pressure.

A further embodiment of the natural cavity location indication device comprises an attachable intermediary apparatus which is adapted to connect between the distal end of the syringe and the proximal end of the probe such as a needle. This intermediary apparatus acts as a visual indicator signalling when the natural cavity has been reached. The visual indicator comprises either an expandable bellows or an expandable diaphragm. As a user advances the probe through the tissue employing the loss of resistance technique, the visual indicator expands upwards corresponding to the increase in applied pressure. Once a drop in pressure is detected, the visual indicator collapses signifying that the natural cavity has been reached.

Further to the aforementioned embodiments, the natural cavity location indication device can be made by injection moulding, vacuum moulding or three-dimensional printing due to the relatively non-complicated geometrical design. In a further aspect, the present invention provides for an injection device for medical use.

The invention may also provide a kit suitable for performing epidural administration or alternative applications according to the invention.

### Brief description of the drawings

Other aspects, advantages and novel features of this invention will become more apparent from the following detailed description considered in connection with the accompanying drawings. However, it should be understood that the drawings are designed as an illustration only and not as a definition of the limits of the invention. In the drawings:
FIG. **1** illustrates a perspective view of a preferred embodiment of a natural cavity location indication device according to the invention, attached to a needle, the device being in an equilibrium position.
FIG. **2** illustrates a perspective view of the device of Figure 1 in a suitably pressurised position.
FIG. **3** illustrates a perspective view of the device shown in Figures 1 and 2 in the equilibrium position, illustrating in hidden detail various internal features.
FIG. **4** illustrates a perspective view of the device shown in Figures 1 and 2 in the pressurised position, illustrating in hidden detail various internal features.
FIG. **5** illustrates a cross-sectional frontal elevation of the preferred embodiment of the device in the pressurised position.
FIG. **6** illustrates a perspective view of an alternative embodiment of a location indication device according to the invention, with a partially curved flange and tapered visual indicator pressure chamber.
FIG. **7** illustrates a perspective view of a further alternative embodiment of a location indication device according to the present invention.
FIG. **8** illustrates a perspective view of a still further embodiment of a location indication device with a radially expanding balloon located towards the distal end of the device.
FIG. **9** illustrates a perspective view of a final embodiment of a location indication device according to the invention with an attachable pressure drop indicator with expandable bellows; and
FIG. **10** illustrates the preferred embodiment of Figs. 1 to 5 of a natural cavity location indication device in the pressurised position advancing toward the epidural space.

### Detailed description of the drawings

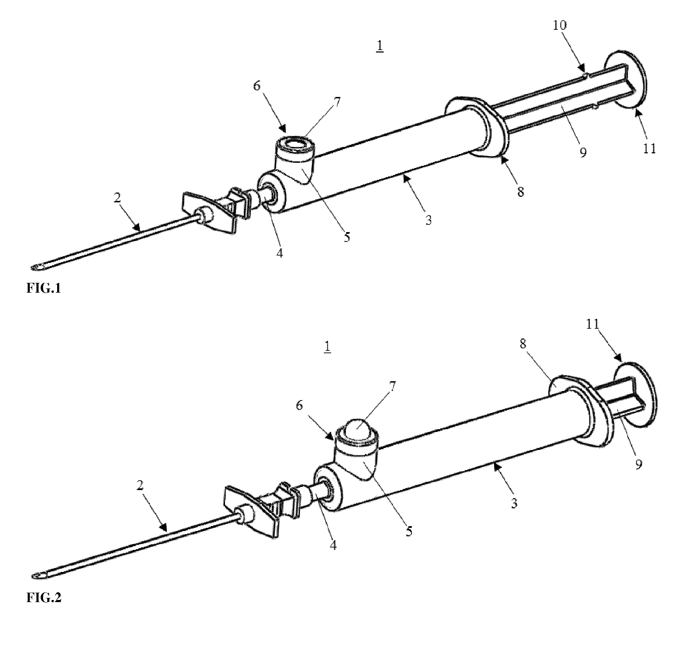

FIGS. **1** and **2** illustrate a natural cavity location indication device **1** connected to a probe in the form of a needle **2** in accordance with a preferred embodiment of the present invention. The device **1** comprises a pressurisable chamber defined by a tubular bodied syringe barrel **3** which connects to the needle **2,** preferably but not essentially via an adapter **4** at a distal end of the syringe barrel **3.**

A pressure applicator in the form of a plunger or piston **9** is slidably received within the syringe barrel **3.** A stop in the form of an extrusion **10** protrudes from a web of the plunger **9** at its proximal end. This extrusion **10** limits the axial displacement of the plunger **9** as it passes through the chamber of the syringe barrel **3.** A handle in the form of a flange **8** is located at the proximal end of the barrel **3** to accommodate the first and middle finger of the user. A circular plane **11** orthogonal to the axis of movement is located at the proximal end of the plunger **9** for receipt of the user's thumb. A visual pressure change indicator **5** is integrated into the distal portion of the barrel **3.** The visual indicator **5** comprises an enclosure in fluid communication with the interior chamber of the syringe barrel 3 at one end and at an opposed end comprises a diaphragm **7** and a cap **6.** The cap **6** secures the perimeter of the diaphragm **7** whilst allowing its centre to bulge outwards through an opening in the cap **6.**

FIG. **1** illustrates the natural cavity location indication device **1** connected to a needle **2** in accordance with a preferred embodiment of the present invention in an equilibrium state. The plunger **9** which is configured as a loss of resistance (LOR) plunger is withdrawn. The needle **2** is first inserted through the skin and into the subcutaneous fat of the patient towards the natural cavity. The syringe barrel **3** is then attached to the needle **2** by means of an adapter **4,** or by any other suitable means. The adapter **4** may take the form of a luer slip or luer lock connection but may also be adapted for a specific epidural connection. The barrel **3** is either completely or partially filled with a fluid, typically air or saline, by withdrawing the plunger **9** from the barrel **3** such as to draw in a volume of said fluid in conventional fashion. The internal diameter of the barrel **3** is at least 5 mm and no more than 20 mm and the volume of fluid drawn into the barrel **3** can range from 2 to 7 ml. Alternative embodiments of the indication device may of course have different dimensions and working volumes.

FIG. **2** illustrates the natural cavity location device **1** connected to the needle **2** in accordance with a preferred embodiment of the present invention, and in a pressurised state. The plunger **9** has been partially advanced creating a positive pressure within the syringe barrel **3** thus providing a corresponding opposing reaction force felt by the user's thumb in applying a compressive force to the plunger **9.** As the needle **2** is advanced through the subcutaneous tissue and a constant or pulsing pressure is applied to the plunger **9,** a positive pressure is created within the barrel **3.** The visual indicator **5** which is integrated with the distal end of the barrel **3,** experiences an equal increase in positive pressure and in doing so causes the elastomeric diaphragm **7** to bulge outwards.

Ideally the visual indicator **5** exhibits a change in colour discerning between the equilibrium and pressurised state. In the preferred embodiment, the visual indicator comprises two diaphragms, one directly overly the other (not shown). The lower diaphragm is hidden from view by the upper diaphragm **7** when the device **1** is in its equilibrium state. One or many apertures or slits (not shown) run through the upper diaphragm **7.** When the enclosure of the visual indicator **5** is pressurised via pressure in the syringe barrel **3,** the lower diaphragm, which preferably comprises a different, contrasting colour relative to the upper diaphragm **7,** bulges upwards through the slits. Once the natural cavity has been reached, the drop in pressure returns the bulging diaphragm to its withdrawn position.

The diaphragm **7** in FIG. **2** retains its bulged profile until the needle tip enters the natural cavity. At which point the sudden drop in pressure causes the diaphragm **7** to immediately collapse and the plunger **9** to advance due to the loss of resistance. The sudden loss in haptic feedback from the plunger **9** and visual collapse of the diaphragm **7** indicates that the natural cavity has been reached. In the application of epidural administration, the introduction of fluid into the epidural space on passing through the ligamentum flavum deflects the dura tissue from the tip of the epidural needle **2.** The volume of fluid introduced depends on the medium. Compressible gas such as air will introduce approximately 2.5 ml whereas the incompressible saline introduces approximately 0.5 ml.

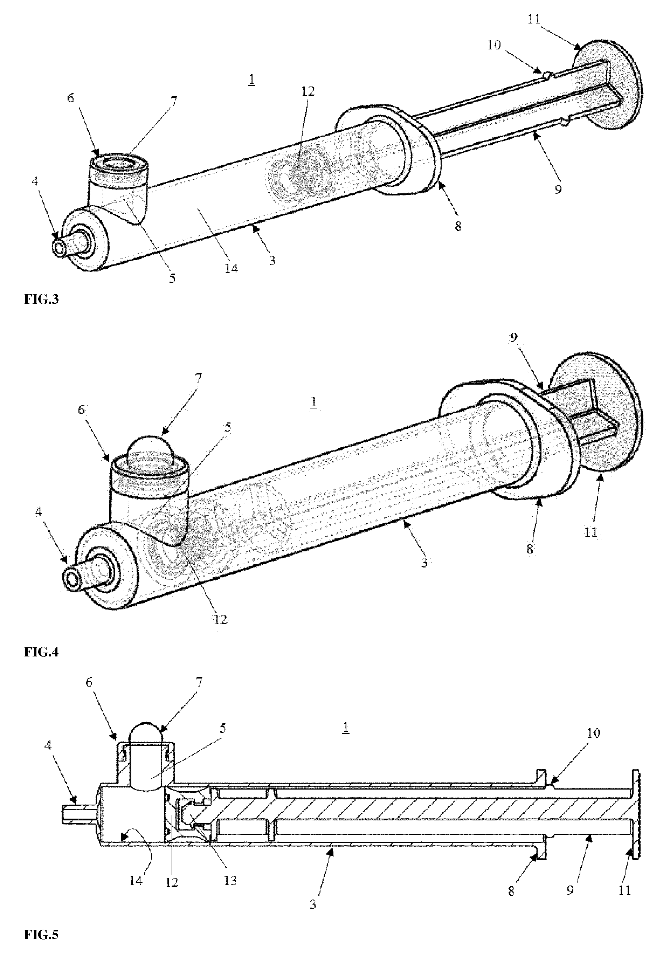

Perspective views of the preferred embodiment of the present invention with hidden detail are illustrated in FIGS. **3** and **4****.** The plunger **9** preferably comprises a shaft with a cross sectional shape of either three of four webbed segments extending from the central axis, the flange **11** located at the proximal end, and a plunger seal **12** located at the distal end. The plunger seal **12** is configured as a LOR plunger cap which reduces the friction between the internal surface of the barrel **3** and the seal **12** whilst ensuring an air tight contact. The plunger seal **12** is made from a soft flexible material, typically silicone.

FIG. **5** illustrates a cross sectional frontal view of the preferred embodiment of the indication device **1.** In this illustration, a chamber **14** within the barrel **3** and visual indicator **5** are pressurised and the diaphragm **7** is fully inflated. The plunger seal **12** is attached to the plunger head **13** on the distal end of the plunger **9** by means of a snap-fit connection. The stopper **10** extends radially outwards from the top of each web on the proximal end of the plunger shaft which prevents the plunger seal **12** from advancing beyond the entrance to the enclosure of the visual indicator **5.** The pressure within the chamber **14** is sufficient to prevent tissue from lodging in the needle as it is advanced towards the natural cavity.

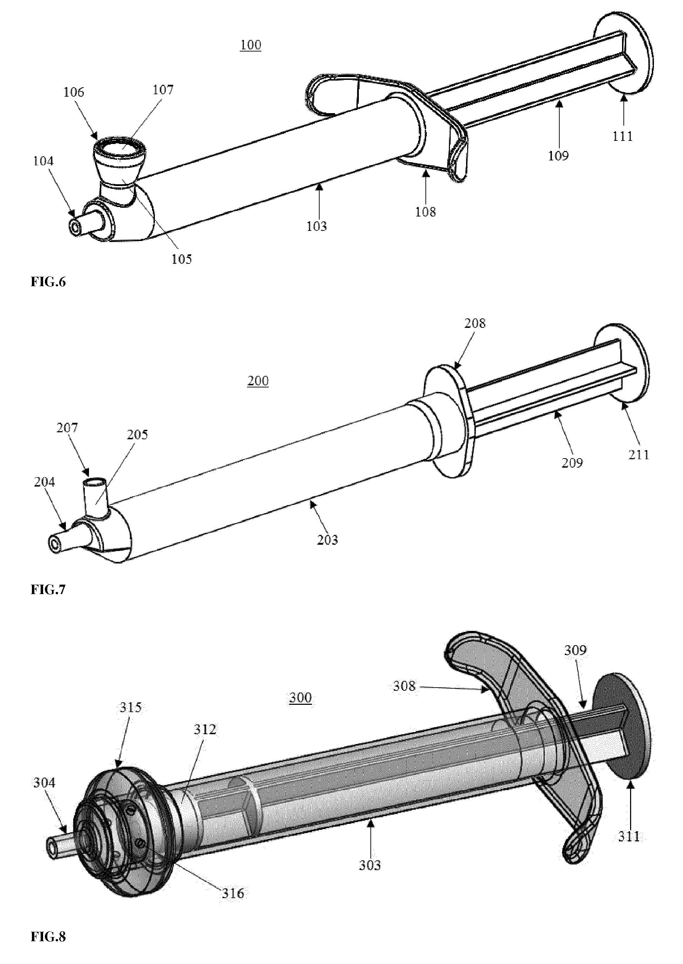

A second embodiment of a natural cavity location device is presented in FIG. **6****,** generally indicated as **100,** in which embodiment like components have been accorded like reference numerals, and unless otherwise stated perform a like function. In this embodiment the indication device **100** comprises an integrated visual pressure change indicator **105** which comprises a tapered enclosure with a smaller diameter intersecting with a syringe barrel **103** and a larger diameter enclosing an expandable diaphragm or membrane **107.** This construction has the added advantage of maintaining a relatively large membrane area upon which the internal positive pressure is applied whilst reducing the area of intersection between the barrel **103** and the visual indicator **105.** A handle in the form of a flange **108** is curved to accommodate the first and second fingers of the user providing a better grip of the syringe barrel **103.**

FIG. **7** illustrates a third embodiment of the present invention, generally indicated as **200,** in which embodiment like components have been accorded like reference numerals, and unless otherwise stated perform a like function. The device **200** comprises a handle in the form of a flange **208** on a proximal end of a syringe barrel **203** which has been orientated 90 degrees relative to the previous embodiments. This allows the user to hold the apparatus **200** with his/her first and second fingers aligned along a horizontal plane. A visual indicator **205** has been reduced in cross sectional area increasing the restoring force in the plunger for the equivalent pressure required to cause a diaphragm **207** to bulge outwards.

A fourth embodiment of an indication device of present invention is illustrated in FIG. **8****,** generally indicated as **300,** in which embodiment like components have been accorded like reference numerals, and unless otherwise stated perform a like function. This embodiment of the device **300** comprises a diaphragm in the form of a radially expanding annular balloon **315** mounted around the external circumference of a syringe barrel **303.** The balloon **315** is located towards the distal end of the apparatus and is inflated through a number of inflation holes **316** cut in a circular array around the circumference of the syringe barrel **303.** As a plunger **309** is advanced, a chamber within the syringe barrel **303** experiences a positive pressure which in turn inflates the balloon **315.** Once the natural cavity has been reached, the internal apparatus pressure drops and the balloon **315** immediately deflates due to the elastic nature of its material, in order to provide the visual indicator to the user that the cavity has been reached.

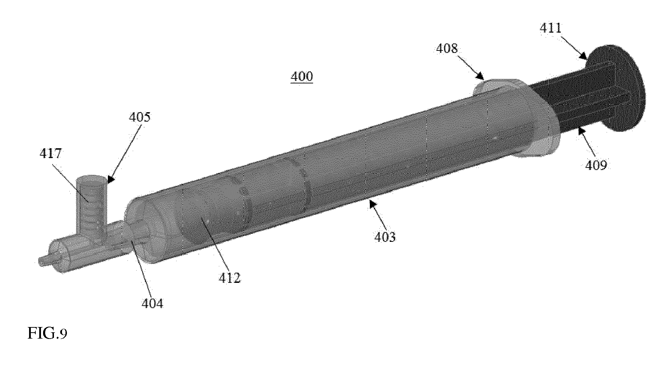

FIG. **9** illustrates a fifth embodiment of the natural cavity location device in accordance with the present invention, generally indicated as **400,** in which embodiment like components have been accorded like reference numerals, and unless otherwise stated perform a like function. Operation of the device **400** is the same as for the above described embodiments, except that a visual pressure change indicator **405** is not integrated with a syringe barrel **403** but is attached by means of an adaptor **404** at a distal end of the syringe barrel **403.** The visual indicator **405** typically comprises a bellows **417** or expandable diaphragm and is positioned between the syringe barrel **403** and a needle. The expansion and contraction of the visual indicator **405** compliments the LOR technique and relates to the positive pressure derived from the force exerted on a pressure applicator in the form of a plunger **409** by the user. In this embodiment, the length of the syringe barrel **403** has been elongated and its diameter has been reduced relative to an existing LOR syringe. This reduction in cross-sectional area extenuates the tactile feeling as the plunger **409** is advanced forward as the applied force is inversely proportional to the cross sectional area. This embodiment is particularly advantageous as the visual indicator **405** may be removed when not required and the modified extenuating LOR syringe **403** may be used independently.

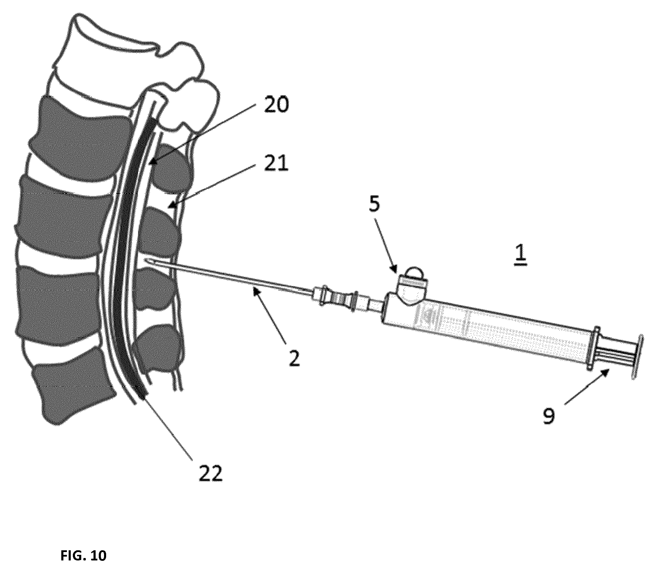

FIG.10 illustrates the natural cavity location indication device **1** as illustrated in Figures 1 to 5, connected to a probe in the form of the needle **2** and being advanced through tissue towards the epidural space. The visual pressure change indicator **5** is pressurised as the plunger **9** is in actuated position. As the distal tip of the needle **2** passes through the interspinous ligament **21,** the tissue pressing up against the needle tip acts as a seal preventing premature deflation of the visual pressure indicator **5** which would otherwise occur through ejection of the anaesthetic or other working fluid contained, under pressure, from the device **1 .** Once the needle tip breaches the ligamentum flavum and enters the epidural space **20** the pressure drop causes the diaphragm of the visual pressure indicator **5** to immediately collapse thus demonstrating that the epidural space has been reached and notifying the user that no further advancement is required. This obviates the possibility of accidentally advancing the needle into the subarachnoid space **22.**

It will therefore be appreciated that the location indication device of the present invention provides an improved device and method for indicating to a user that a natural body cavity has been reached in order to allow a working fluid such as an anaesthetic to be injected into that natural body cavity.

## Claims

1. An indication device which provides both visual and haptic feedback in signalling entry of a probe into a natural cavity, the device comprising;
a pressurisable chamber having at least an outlet from which a working fluid may be dispensed;
a manually actuatable pressure applicator operable to alter the pressure within the chamber; and
a visual pressure change indicator in fluid communication with the chamber and comprising at least one pressure expandable diaphragm.

2. An indication device according to claim 1 in which the at least one expandable diaphragm is arranged to bulge outwards to signify when the chamber is suitably pressurised.

3. An indication device according to claim 1 or 2 in which the visual pressure change indicator comprises an enclosure having an entry in fluid communication with the chamber, and an opening over which the at least one expandable diaphragm provides a fluid tight covering.

4. An indication device according to any preceding claim in which the visual pressure change indicator is adapted to change colour in response to a predefined pressure change within the chamber.

5. An indication device according to any preceding claim in which the visual pressure change indicator comprises an first diaphragm having one or more apertures therein; and a second diaphragm underlying the first diaphragm and adapted to protrude through the one or more apertures in said upper diaphragm when the chamber is suitably pressurised.

6. An indication device according to claim 5 in which the first and second diaphragms are of contrasting colour.

7. An indication device according to any preceding claim in which the outlet defines a connection port adapted to receive a hollow probe in fluid communication with the chamber.

8. An indication device according to any preceding claim in which the chamber is defined by a syringe barrel.

9. An indication device according to claim 8 in which the pressure change indicator is formed integrally with the syringe barrel.

10. An indication device according to any of claims 8 to 10 comprising a handle on the syringe barrel.

11. An indication device according to any of claims 8 to 10 in which the pressure applicator comprises a plunger slidably received within the syringe barrel.

12. An indication device according to claim 11 in which the plunger is adapted to operate as a loss of resistance plunger.

13. An indication device according to claim 11 or 12 comprising a stop operable to limit the axial displacement of the plunger within the syringe barrel.

14. An indication device according to any of claims 8 to 13 wherein the internal diameter of said syringe barrel is no less than 5 mm and no more than 20 mm.

15. A method of indicating the location of a natural cavity comprising the steps of:
pressurising a chamber having at least an outlet from which a working fluid may be dispensed through a probe secured in fluid communication to the outlet by manually actuating a pressure applicator in operative association with the chamber;
advancing the probe through tissue towards the natural cavity while applying a compressive force to the pressure applicator until a visual change occurs in a visual pressure change indicator in fluid communication with the chamber and a loss in resistance occurs to the application of force to the pressure applicator as the probe enters the cavity.
